# EUROPÄISCHE PATENTSCHRIFT

(11) **EP 2 046 276 B1**
(45) Veröffentlichungstag und Bekanntmachung des Hinweises auf die Patenterteilung: **30.12.2015**
(21) Anmeldenummer: 07785882.7
(22) Anmeldetag: 03.07.2007
(51) Int. Cl.: A61K 8/34, A61K 8/67, A61Q 17/04, A61Q 19/08

(54) **KOSMETISCHE ODER DERMATOLOGISCHE ZUBEREITUNGEN MIT EINEM GEHALT AN FOLSÄURE UND EINEM ODER MEHREREN POLYOLEN**
COSMETIC OR DERMATOLOGICAL PREPARATIONS CONTAINING FOLIC ACID AND ONE OR MORE POLYOLS
PRÉPARATIONS COSMÉTIQUES OU DERMATOLOGIQUES CONTENANT DE L'ACIDE FOLIQUE ET UN OU PLUSIEURS POLYOLS

(30) Priorität: 13.07.2006 DE 102006032413
(43) Veröffentlichungstag der Anmeldung: 15.04.2009
(73) Patentinhaber: Beiersdorf AG, 20253 Hamburg (DE)
(72) Erfinder: DIPPE, Rixa, 20255 Hamburg (DE); DRUCKS, Anja, 22399 Hamburg (DE); MEDING, Christel, 25462 Rellingen (DE); NIELSEN, Jens, 24558 Henstedt-Ulzburg (DE); TREU, Jens, 22864 Norderstedt (DE)
(86) Internationale Anmeldenummer: PCT/EP2007/005870
(87) Internationale Veröffentlichungsnummer: WO 2008/006485

(56) Entgegenhaltungen:
- EP-A- 0 311 259
- EP-A- 0 652 012
- EP-A- 1 214 927
- EP-A- 1 609 462
- WO-A-2004/054936
- WO-A-2005/063184
- WO-A-2007/014644
- DE-A1- 4 341 001
- DE-A1- 10 300 782
- GB-A- 1 289 096
- US-A1- 2006 120 967
- DATABASE CA [Online] CHEMICAL ABSTRACTS SERVICE, COLUMBUS, OHIO, US; 25. April 2002 (2002-04-25), SHINSHI, KAZUNORI ET AL: "Aqueous solutions containing vitamin B" XP002450774 gefunden im STN Database accession no. 136:284437 & JP 2002 097141 A (NIKKEN CHEMICALS CO., LTD., JAPAN; NIKKEN KASEI K. K.) 2. April 2002 (2002-04-02)

## Beschreibung

Die vorliegende Erfindung betrifft kosmetische oder dermatologische Zubereitungen mit einem Gehalt an Folsäure und einem oder mehreren Polyolen.

Die Haut ist einer Vielzahl von Umwelteinflüssen ausgesetzt, die zu ihrer Schädigung führen können. Neben Lipiden und Proteinen ist dabei vor allem die DNA betroffen.

Die schädigende Wirkung des ultravioletten Teils der Sonnenstrahlung auf die Haut ist allgemein bekannt. Während Strahlen mit einer Wellenlänge, die kleiner als 290 nm ist (der so genannte UVC-Bereich), von der Ozonschicht in der Erdatmosphäre absorbiert werden, verursachen Strahlen im Bereich zwischen 290 nm und 320 nm, dem so genannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, des sogenannten UVA-Bereich, ist es wichtig, Filtersubstanzen zur Verfügung zu haben, da dessen Strahlen Reaktionen bei lichtempfindlicher Haut hervorrufen können. Es ist erwiesen, dass UVA-Strahlung zu einer Schädigung der elastischen und kollagenen Fasern des Bindegewebes führt, was die Haut vorzeitig altern läßt, und dass sie als Ursache zahlreicher phototoxischer und photoallergischer Reaktionen zu sehen ist. Der schädigende Einfluß der UVB-Strahlung kann durch UVA-Strahlung verstärkt werden.

Insbesondere Hautzellen erhalten durch häufige UV-Exposition einen hohen Anteil an DNA-Schäden und benötigen deshalb besonders effiziente und leistungsfähige Reparatursysteme. Nur effiziente hauteigene DNA-Reparatursysteme sind in der Lage, Schäden schnell zu beseitigen, wodurch Hautveränderungen und ein frühzeitiges Auftreten von Hautalterungserscheinungen verhindert werden können. Effiziente DNA-Reparatur trägt damit entscheidend zum Erhalt einer gesunden, vitalen Haut bei. Stimulierung und Unterstützung hauteigener Reparatursysteme durch kosmetisch-dermatologische Inhaltsstoffe sind deshalb sehr wichtig.

Die angesichts der großen Anzahl Nucleotide, die pro Zellteilung kopiert werden müssen, notwendige hohe Zuverlässigkeit bei der DNA-Replikation wird einerseits durch die Spezifität der DNA-Polymerasen, die an der Replikation beteiligt sind (Replicasen), andererseits durch deren zusätzlich vorhandene Exonucleaseaktivität garantiert: Sie katalysieren das Herausschneiden unpassender Nucleotide und deren Ersatz durch die richtigen sofort nach der Neusynthese. Den durch chemische Einwirkung entstehenden Schäden (durch Mutagene, von denen einige sich zwischen zwei übereinanderliegende Basenpaare der Doppelhelix einschieben können) sowie den von Strahlungseinwirkung herrührenden Veränderungen (wobei z. B. zwei übereinanderliegende Thymin-Reste dimerisieren können) helfen Reparatur-Enzyme (darunter: Endonucleasen, Polymerasen, Ligasen) ab, die unpassende Nucleotide wieder aus den DNA herausschneiden und ersetzen. Einige dieser Reparatursysteme sind induzierbar, d. h. sie werden erst bei Bedarf synthetisiert. Die Frage, welcher Strang die Original-Information enthält und welcher falsch synthetisiert wurde, wird vom Reparaturenzym offenbar anhand des Methylierungszustands entschieden.

Durch Exposition zu Umwelteinflüssen (z.B. UV-Strahlung, chemische und physikalische Faktoren) entstehen Schäden an den DNA-Molekülen der Zellen des Körpers, die jedoch durch zelleigene DNA-Reparaturmechanismen behoben werden können. Dabei erfolgt zuerst die Erkennung des DNA-Schadens und dann die Reparatur des vorliegenden Schadens. Werden die Schäden nicht vollständig kurz nach ihrer Entstehung durch Reparaturprozesse beseitigt, reichern sich diese in Form von permanenten DNA-Schäden in den Zellen an und werden nachfolgend an die Tochterzellen weitergegeben. Diese permanenten Schäden besitzen insbesondere durch mögliche Spätfolgen (schrittweise Funktionsausfälle) ein hohes Schadenspotential und spielen einen immer größere Rolle in Alterungsprozessen und bei der Schadensauslösung bereits im Kindesalter.

Aufgabe der vorliegenden Erfindung war es mithin, den Übelständen des Standes der Technik Abhilfe zu schaffen. Insbesondere sollten Wirkstoffe und Zubereitungen zur Verfügung gestellt werden, die eine sichere Prophylaxe von Schäden an der hauteigenen DNA gewährleisten und/oder in einer besonderen Ausführungsform zur Reparatur bereits eingetretener Schäden an der hauteigenen DNA dienen sollte.

Folsäure weist folgende Struktur auf:

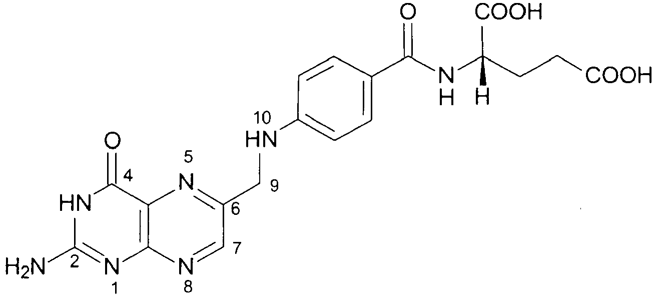

Folsäure kommt in Leber, Niere, Hefe, Pilzen, Getreide und grünen Blättern, vorwiegend als Konjugat mit Poly-γ-L-glutaminsäure (Pteroylpolyglutaminsäuren) vor. Sie wurde als Wuchsstoff für verschiedene Mikroorganismen entdeckt und hat für den menschlichen Organismus Vitamincharakter. Der Bedarf des erwachsenen Menschen beträgt etwa 200 µg je Tag an bioverfügbarem Folat.

Folsäure steht im Organismus im Gleichgewicht mit 7,8-Dihydrofolsäure (H₂Folat; alte Abkürzung: FH₂) unter Beteiligung von Nicotinamid-Adenin-Dinucleotid-Phosphat und des Enzyms Dihydrofolat-Reduktase. H₂Folat wiederum entsteht in Pflanzen und einigen Mikroorganismen über mehrere Zwischenstufen aus Guanosin-5'-triphosphat (Guanosinphosphate) und setzt sich mit Hilfe der Dihydrofolat-Reduktase zu (6S)-5,6,7,8-Tetrahydrofolsäure um, der eigentlichen physiologisch wirksamen Form der Folsäure.

### 7,8-Dihydrofolsäure hat folgende chemische Struktur:

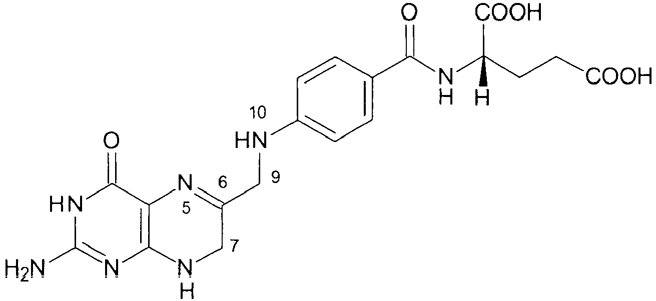

### (6S)-5,6,7,8-Tetrahydrofolsäure hat folgende chemische Struktur:

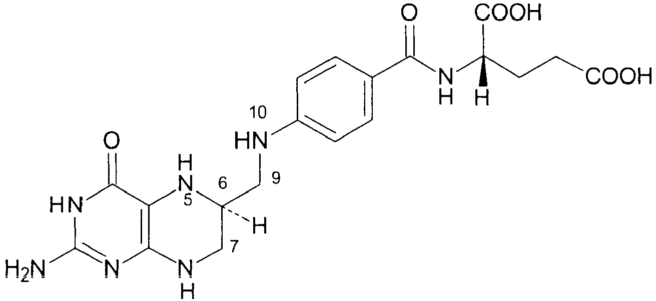

Unter dem Begriff der Derivate der Folsäure sind insbesondere die vorgenannte Dihydrofolsäure und die Tetrahydrofolsäure zu verstehen.

Folsäure ist ein Wirkstoff, der nur bei einem pH Wert von > 6,5 gelöst werden kann. Häufig besteht jedoch der Wunsch, eine Emulsion unabhängig von pH Wert / bei niedrigerem pH Wert herzustellen.

EP 1 214 927 beschreibt die Wirkung der Folsäure bei DNA Repratur Prozessen. Es beschreibt auch topische Zubereitungen enthaltend Folsäure in Kombination mit Glycerin, Butylenglycol oder Panthenol Erstaunlicherweise werden diese Aufgaben gelöst durch kosmetische oder dermatologische Zubereitungen mit einem Gehalt an Folsäure und/oder deren Derivate und einem oder mehreren Polyolen.

Erfindungsgemäß weisen Folsäure und/oder deren Derivate ein hohes Potential zur Reparatur geschädigter DNA auf. Ferner bieten Folsäure und/oder deren Derivate wirksame Prophylaxe gegen DNA-Schäden, insbesondere solche Schäden, die durch UV-Strahlung hervorgerufen werden.

Erfindungsgemäß wird eine vom pH-Wert unabhängige Löslichkeit von Folsäure erreicht. Vorteilhafte Poylyole sind 1,2-Butandiol, 2,3-butandiol 2,3-Dimethyl-2,3-butandiol, 1,2-Octandiol 1,2-Hexandiol.

Das Verhältnis von Folsäure zu Polyol (bzw. der Gesamtmenge an Polyolen) sollte 1:10 bis 1:1000 betragen.

Die kosmetischen oder dermatologischen Zubereitungen können erfindungsgemäß wie üblich zusammengesetzt sein und zur Behandlung, der Pflege und der Reinigung der Haut und/oder der Haare und als Schminkprodukt in der dekorativen Kosmetik dienen.

Sie enthalten bevorzugt 0,001 Gew.-% bis 10 Gew.-%, bevorzugt 0,05 Gew.-% bis 5 Gew.-%, insbesondere 0,1 - 2,0 Gew.-%, bezogen auf das Gesamtgewicht der Zubereitungen, an Folsäure und/oder deren Derivaten.

Die kosmetischen oder dermatologischen Zubereitungen enthalten ferner bevorzugt 0,001 Gew.-% bis 30 Gew.-%, bevorzugt 0,05 Gew.-% bis 10 Gew.-%, insbesondere 0,1 - 2,0 Gew.-%, bezogen auf das Gesamtgewicht der Zubereitungen, an einem oder mehreren Polyolen.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/O/W), ein Gel, einen festen Stift, eine Salbe oder auch ein Aerosol darstellen. Es ist auch erfindugsgemäß vorteilhaft, Folsäure und/oder deren Derivate in verkapselter Form darzureichen, z.B. in Kollagenmatrices und anderen üblichen Verkapselungsmaterialien, z.B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt.

Es ist auch möglich und vorteilhaft im Sinne der vorliegenden Erfindung, Folsäure und/oder deren Derivate in wäßrige Systeme bzw. Tensidzubereitungen zur Reinigung der Haut und der Haare einzufügen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Siliconderivate.

Insbesondere können Folsäure und/oder deren Derivate erfindungsgemäß auch mit anderen Antioxidantien und/oder Radikalfängern kombiniert werden.

Die Menge an Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Siliconöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Als Treibmittel für erfindungsgemäße, aus Aerosolbehältern versprühbare Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, Gewichtsprozente, auf das Gewicht und die Gesamtmenge bzw. auf das Gesamtgewicht der Zubereitungen bezogen.

| **OW-Emulsionen** | **1** | **2** | **3** | **4** |
|---|---|---|---|---|
| Natriumhydroxid | 0,2 | 0,6 | 0,15 | 0,5 |
| Na₃HEDTA | 0,5 | 1 | 0,5 | 1 |
| Glycerin | 10 | 7,5 | 7,5 | 5 |
| Glycerylstearatcitrat | 2 | 2,5 | | |
| PEG-40 Stearat | | | 1,0 | 0,7 |
| Glvcerylstearat | | | 2,5 | 2,5 |
| Stearylalkohol | 2 | 3 | 1 | 1,5 |
| Cetylalkohol | 2 | | 1 | |
| Carbomer | 0,1 | | 0,3 | 0,1 |
| Acrylat/C10-30 Alkylacrylat Crosspolymer | 0,2 | 0,3 | | 0,2 |
| Myristylmyristat | | | | |
| C12-15 Alkyl Benzoate | 2 | 4 | 2 | 4 |
| Octyldodecanol | 2 | | | 2 |
| Caprylsäure/Caprinsäuretriglyceride | 1 | 2 | 1 | 2 |
| Cera Microcristallina + Paraffinum Liquidum | 3 | | 2 | |
| Butyrospermum Parkii | | 3 | 3 | |
| Dimethicone | 1 | 2 | 1 | 3 |
| Cyclomethicone | 3 | 4 | 1,6 | 3 |
| Methylparaben + Ethylparaben + Propylparaben | 0,4 | 0,5 | | 0,4 |
| Phenoxyethanol | 0,4 | 0,6 | 0,4 | 0,5 |
| Ethylhexylglycerin | 0,5 | | | 0,3 |
| 1,2-Octandiol | | | 0,25 | |
| 1,2-Hexanediol | | 0,5 | | 0,5 |
| Methylpropanediol | 4 | 4 | 2 | 2 |
| Butylmethoxydibenzoylmethan | | 2 | | 3 |
| Ethylhexylmethoxycinnamat + BHT | | 5 | | 7 |
| Phenylbenzimidazolsulfonsäure | | 2 | | 2 |
| TiO₂ + Trimethoxycaprylylsilane | | 2 | 1,5 | 0,5 |
| Tapiokastärke + Wasser | 2 | | 1 | 3 |
| Talkum | 1,5 | 2 | 0,5 | |
| Tocopheryl Acetate | 0,5 | 1 | 1 | 1,5 |
| Folsäure | 0,05 | 0,1 | 0,025 | 0,1 |
| Kreatin | 0,2 | 0,5 | 0,2 | 0,1 |
| Aqua | Ad 100 | Ad 100 | Ad 100 | Ad 100 |

## Patentansprüche

1. Kosmetische oder dermatologische Zubereitungen in Form von Emulsionen mit einem Gehalt an Folsäure und einem oder mehreren Polyolen, gewählt aus der Gruppe 1,2-Butandiol, 2,3-Butandiol, 2,3-Dimethyl-2,3-butandiol, 1,2-Octandiol, 1,2-Hexandiol, wobei die Zubereitungen einen pH ≤ 6,5 aufweisen.

2. Zubereitungen nach Anspruch 1, **dadurch gekennzeichnet, daß** sie 0,001 Gew.-% bis 10 Gew.-%, bevorzugt 0,01 Gew.-% bis 1,0 Gew.-%, insbesondere 0,05 - 0,5 Gew.-%, bezogen auf das Gesamtgewicht der Zubereitungen, an Folsäure enthalten.

3. Zubereitungen nach Anspruch 1 oder 2, **dadurch gekennzeichnet, daß** sie 0,001 Gew.-% bis 30 Gew.-%, bevorzugt 0,05 Gew.-% bis 10 Gew.-%, insbesondere 0,1 - 2,0 Gew.-%, bezogen auf das Gesamtgewicht der Zubereitungen, an einem oder mehreren Polyolen aufweisen.

4. Zubereitungen nach einem der vorstehenden Ansprüche, **dadurch gekennzeichnet, daß** das Verhältnis von Folsäure zu Polyol (bzw. der Gesamtmenge an Polyolen) aus dem Bereich 1:10 bis 1:1000 gewählt wird.

## Claims

1. Cosmetic or dermatological preparations in the form of emulsions with a content of folic acid and one or more polyols selected from the group 1,2-butanediol, 2,3-butanediol, 2,3-dimethyl-2,3-butanediol, 1,2-octanediol, 1,2-hexanediol, with the preparations having a pH ≤ 6.5.

2. Preparations according to Claim 1, **characterized in that** they comprise 0.001% by weight to 10% by weight, preferably 0.01 % by weight to 1.0% by weight, in particular 0.05-0.5% by weight, based on the total weight of the preparations, of folic acid.

3. Preparations according to Claim 1 or 2, **characterized in that** they have 0.001% by weight to 30% by weight, preferably 0.05% by weight to 10% by weight, in particular 0.1-2.0% by weight, based on the total weight of the preparations, of one or more polyols.

4. Preparations according to one of the preceding claims, **characterized in that** the ratio of folic acid to polyol (or the total amount of polyols) is selected from the range 1:10 to 1:1000.

## Revendications

1. Préparations cosmétiques ou dermatologiques sous forme d'émulsions ayant une teneur en acide folique et en un ou plusieurs polyols, choisis dans le groupe constitué par le 1,2-butanediol, le 2,3-butanediol, le 2,3-diméthyl-2,3-butanediol, le 1,2-octanediol, le 1,2-hexanediol, les préparations présentant un pH ≤ 6,5.

2. Préparations selon la revendication 1, **caractérisées en ce qu'**elles contiennent 0,001 % en poids à 10 % en poids, de préférence 0,01 % en poids à 1,0 % en poids, en particulier 0,05 - 0,5 % en poids, par rapport au poids total des préparations, d'acide folique.

3. Préparations selon la revendication 1 ou 2, **caractérisées en ce qu'**elles contiennent 0,001 % en poids à 30 % en poids, de préférence 0,05 % en poids à 10 % en poids, en particulier 0,1 - 2,0 % en poids, par rapport au poids total des préparations, d'un ou de plusieurs polyols.

4. Préparations selon l'une quelconque des revendications précédentes, **caractérisées en ce que** le rapport de l'acide folique au polyol (ou à la quantité totale de polyols) est choisi dans la plage de 1:10 à 1:1000.
